# EUROPEAN PATENT SPECIFICATION

(11) **EP 3 525 762 B1**
(45) Date of publication and mention of the grant of the patent: **23.06.2021**
(21) Application number: 17787333.8
(22) Date of filing: 12.10.2017
(51) Int. Cl.: A61K 9/16, A61K 9/20, A61K 31/4365

(54) **PHARMACEUTICAL COMPOSITION COMPRISING PRASUGREL BESYLATE**
PHARMAZEUTISCHE ZUSAMMENSETZUNG MIT PRASUGREL-BESYLAT
COMPOSITION PHARMACEUTIQUE COMPRENANT DU BÉSYLATE DE PRASUGREL

(30) Priority: 12.10.2016 GR 20160100533
(43) Date of publication of application: 21.08.2019
(73) Proprietor: Pharmathen S.A., 15351 Pallini Attikis (GR)
(72) Inventor: KARAVAS, Evangelos, 15351 Attikis (GR); KOUTRIS, Efthymios, 15351 Attikis (GR); SAMARA, Vasiliki, 15351 Attikis (GR); KOUTRI, Ioanna, 15351 Attikis (GR); KALASKANI, Anastasia, 15351 Attikis (GR); KIZIRIDI, Christina, 15351 Attikis (GR); ABATZIS, Morfis, 15351 Attikis (GR); TSILIOUKA, Katerina, 15351 Attikis (GR)
(86) International application number: PCT/EP2017/025306
(87) International publication number: WO 2018/068898

(56) References cited:
- EP-A1- 2 377 520
- EP-A1- 2 564 847
- EP-A1- 2 722 036
- EP-A2- 2 409 685
- WO-A1-2009/098142
- WO-A1-2010/094471
- US-A1- 2013 035 355
- US-A1- 2015 182 457

## Description

### TECHNICAL FIELD OF THE INVENTION

This present invention belongs to the technical field of pharmaceutical formulation. In particular, this invention relates to an immediate release formulation of Prasugrel besylate for oral administration and a process for preparing such formulation.

### BACKGROUND OF THE INVENTION

Prasugrel is a member of the thienopyridine class of antiplatelet agents. Prasugrel has a distinct chemical structure that permits efficient conversion to its active metabolite, R-138727, that irreversibly inhibits platelet activation and aggregation mediated by the P₂Y₁₂ active adenosine diphosphate (ADP) receptor.

The chemical name of prasugrel is 5-[(1*RS*)-2-cyclopropyl-1-(2-fluorophenyl)-2-oxoethyl]-4, 5, 6, 7-tetrahydrothieno[3,2-c]pyridin-2-yl acetate. Prasugrel hydrochloride is marketed under the trademark Effient® in 5 or 10 mg strengths. The tablets are commercially supplied in blister packaging.

Formulation development of pharmaceutical composition containing prasugrel or salts thereof is challenging due to the poor chemical stability and the poor water solubility of the active pharmaceutical ingredient (API).

Poor chemical stability leads to degradation of the active ingredient and results in reduced effectiveness or treatment failure. Thus, careful selection of excipients is one of a key factor in developing finished dosage forms with good safety.

Prasugrel is a poor water soluble compound. The solubility of prasugrel in a wide range of pH buffer solutions shows that its solubility is high in acidic media and decreases with the increase in pH from neutral to alkali.

Selection of the API crystalline form and proper physical properties is another important factor in developing finished dosage forms with desired dissolution behavior.

Pharmaceutical compositions comprising prasugrel free base or salts thereof as the single drug substance have been disclosed, for example, in WO 2006/135605, WO 2008/073759, WO 2011/092720, WO 2013/150322, and WO 2015/103230. However, the prior art has encountered substantial difficulty in the production of the solid oral formulations with appropriate physical characteristics and stability, especially when Prasugrel is in the dosage form as salt other than hydrochloride.

Therefore, although each of the above patents represents an attempt to overcome the poor stability and poor water solubility problems associated with pharmaceutical compositions comprising prasugrel or pharmaceutically acceptable salts thereof, there still exists a need for improving the stability and therapeutic efficacy of such pharmaceutical compositions.

### SUMMARY OF THE INVENTION

It is, therefore, an object of the present invention to provide a pharmaceutical composition for oral administration of prasugrel besylate, which overcomes the deficiencies of the prior art and provides an effective and stable dosage form.

The present invention aims at developing a formulation that not only matches the physical and chemical attributes of the reference product but also is bioavailable and with sufficient self-life.

A major object of the present invention is the selection of the optimal combination of pharmaceutical acceptable excipients, the effective drug substance particle size distribution and the method of preparation of final product in order to achieve the appropriate dissolution profile and stability for the finished dosage form. Said dosage form affords predictable and reproducible drug release rates in order to achieve better treatment to a patient.

A further approach of the present invention is to provide a tablet composition for oral administration comprising Prasugrel besylate which is manufactured through a fast, simple and cost-effective process.

A major aspect of the present invention is to provide a pharmaceutical formulation that can enhance the solubility of Prasugrel besylate

Accordingly, the present invention provides a pharmaceutical composition comprising prasugrel besylate having particle size distribution of D₉₀ below 60µm and a surfactant in amount 3-8 wt%, in which said surfactant is selected from sodium lauryl sulfate (SLS) and soluplus®.

The pharmaceutical composition according to the present invention further comprises mannitol as a diluent, microcrystalline cellulose as a filler, croscarmellose sodium as a disintegrant, hypromellose as a binder, colloidal silicon dioxide as glidant and magnesium stearate as a lubricant.

Other objects and advantages of the present invention will become apparent to those skilled in the art in view of the following detailed description.

### DETAILED DESCRIPTION OF THE INVENTION

For the purposes of the present invention, a pharmaceutical composition comprising an active ingredient is considered to be "stable" if said ingredient degrades less or more slowly than it does on its own and/or in known pharmaceutical compositions.

As already mentioned the main object of the present invention is to provide a stable pharmaceutical composition of Prasugrel besylate for oral administration that is simple to manufacture, bioavailable, cost effective and possesses good pharmacotechnical properties.

An important parameter to achieve desired concentration of drug in systemic circulation for the anticipated pharmacological response (bioavailability) is solubility.

Solubility is the phenomenon of dissolution of solute in solvent to give a homogenous system. Prasugrel besylate, as already explained, possess variable solubility depending on pH value. Solubility enhancement is a major challenge for formulation development. Any drug to be absorbed must be present in the form of solution at the site of absorption. Various techniques are used for the enhancement of the solubility which include physical and chemical modifications of drug and other methods like particle size reduction, crystal engineering, salt formation, solid dispersion, use of surfactant, complexation, and addition of alkalizing or acidifying agents. Selection of solubility improving method depends on drug property, site of absorption, and required dosage form characteristics.

It has been surprisingly found that the object of the present invention can be achieved by employing SLS or Soluplus®, in the composition. SLS and Soluplus® are effective in enhancing the stability and the solubility of prasugrel besylate.

Sodium lauryl sulfate is an anionic surfactant freely soluble in water. It is widely used in oral and topical pharmaceutical formulations as a detergent and wetting agent effective in both alkaline and acidic conditions.

Soluplus® is a graft copolymer comprised of polyethylene glycol, polyvinyl acetate and polyvinyl caprolactam. Even though Soluplus® was developed as a matrix polymer for solid solutions, it is also capable of solubilizing poorly soluble drugs in aqueous media.

It has also been found that the amount of the surfactant has an impact on the dissolution rate. The use of 3 wt% up to 8 wt% resulted in increased solubility. The target of higher than 85% dissolution at 30 minutes was achieved.

The particle size of the API is another parameter that can affect its solubility. The specific surface area is increased with decreasing particle size of the drug, resulting in an increase in dissolution rate. In most circumstances the dissolution rate of poorly soluble drugs is strongly related to the particle size distribution and thus the dissolution profile of the final product.

In order for a drug to have its effect after oral administration it must go into solution and then diffuse through the gut wall into the body. The first step in that process is the disintegration of the dosage form followed by dissolution of the active ingredient. One way to increase dissolution rate is to increase the surface available for dissolution by reducing particle size. For prasugrel besylate crystalline API, we found that a particle size distribution of D90 below 60µm is suitable to ensure good dissolution rate.

The pharmaceutical compositions of the present invention may also contain one or more additional formulation excipients such as diluents, disintegrants, binders, lubricants, provided that they are compatible with the active ingredient of the composition, so that they do not interfere with it in the composition and in order to increase the stability of the drug and the self-life of the pharmaceutical product.

Diluents increase the bulk of a solid pharmaceutical composition, and may make a pharmaceutical dosage form easier for the patient and care giver to handle. Diluents for solid compositions include, for example, microcrystalline cellulose (MCC), dextrose, fructose, mannitol, maltodextrin, maltitol, lactose.

The dissolution rate of a compacted solid pharmaceutical composition in the patient's stomach may be increased by the addition of a disintegrant to the composition. Disintegrants include sodium starch glycolate, alginic acid, carboxymethylcellulose sodium, croscarmelose sodium, colloidal silicon dioxide (aerosil).

Solid pharmaceutical compositions that are compacted into a dosage form, such as a tablet, may include excipients whose function include helping to bind the active ingredient and other excipients together after compression. Binders for solid pharmaceutical compositions include hydroxyethyl cellulose, methylcellulose, hydroxypropyl cellulose (HPC), polydextrose, polyethylene oxide, povidone.

When a dosage form such as a tablet is made by the compaction of a powdered composition, the composition is subjected to pressure from a punch and dye. Some excipients and active ingredients have a tendency to adhere to the surfaces of the punch and dye, which can cause surface irregularities to the product. A lubricant can be added to the composition to reduce adhesion and ease the release of the product from the dye. Lubricants include talc, magnesium stearate, calcium stearate, glyceryl behenate.

Two polymorphic forms of prasugrel besylate are described in the literature, namely form P and form M. Crystalline Form M of Prasugrel besylate is characterized by the Powder X-ray diffraction having characteristic peaks at 9.34, 17.41, 20.01, 24.03, 25.15 (±) 0.2° degree 2-theta. Crystalline Form P of Prasugrel besylate is characterized by the Powder X-ray diffraction having characteristic peaks at 7.1, 8.15, 9.2, 10.24, 11.03, 13.25, 14.68, 16.05, 19.27, 21.51, 23.21 (±) 0.2° degree 2-theta. The selected crystalline form in the present invention was form P but similar results are to be expected with crystalline form M.

Manufacturing processes such as dry granulation, wet granulation with ETOH and Acetone can be used to produce the solid oral dosage form of the composition of the present invention.

The following examples illustrate preferred embodiments in accordance with the present invention

### EXAMPLES

### Example 1:

The performance of four different surfactants was evaluated in compositions prepared by dry granulation. The manufacturing process followed for the four formulations comprised the following steps:
- Sifting the raw materials
- Blending API with, internal phase excipients
- Lubrication with Magnesium stearate
- Compression

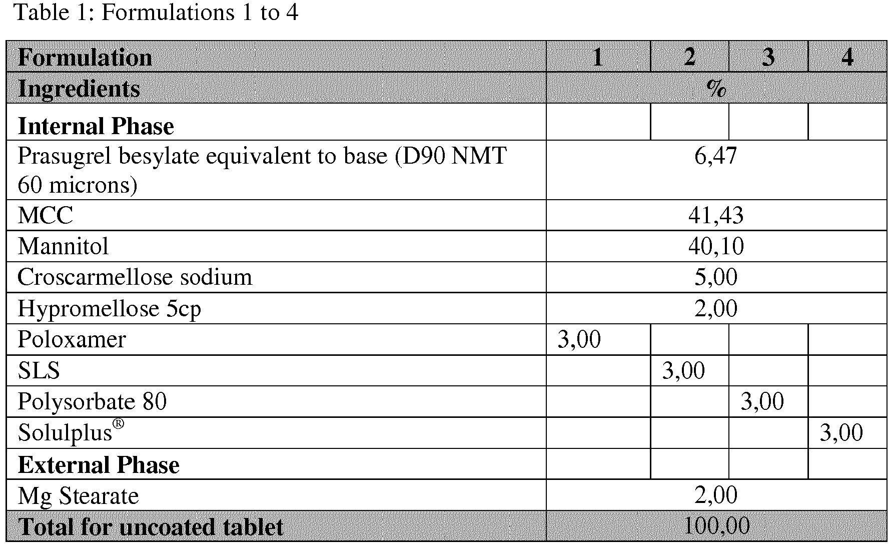

The release of the active ingredient from formulations 1 to 4 was evaluated by performing dissolution testing USP app. II at 75 r.p.m. using a citrate-Phosphate buffer with pH value of 4.

**Table 2: Dissolution profile of formulations 1 to 4**

| **Time interval (min)** | **% Dissolved** | | | |
|---|---|---|---|---|
| | **1** | **2** | **3** | **4** |
| 5 | 40,26 | 36,39 | 36,76 | 39,04 |
| 10 | 47,31 | 49,47 | 44,53 | 52,66 |
| 15 | 48,52 | 56,36 | 47,63 | 60,42 |
| 20 | 50,93 | 59,89 | 50,85 | 64,57 |
| 30 | 57,35 | 65,56 | 55,63 | 68,69 |
| 45 | 64,06 | 70,15 | 59,78 | 71,54 |

The results present a higher release rate for formulations 2 and 4 but not as high as the required for an immediate release dosage form.

### Example 2:

SLS was tested, as one of the two surfactants having better results, in various amounts and using a wet granulation process.

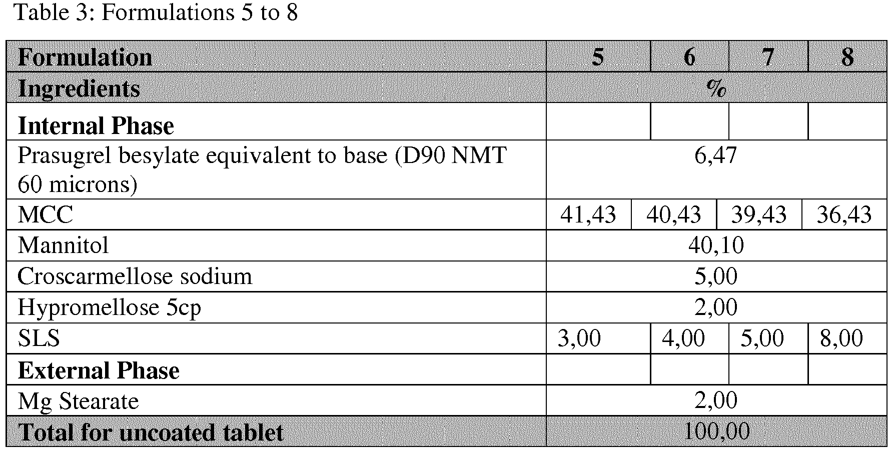

The manufacturing process of formulations 5 to 8 includes wet granulation of internal phase ingredients. The preparation steps followed are presented below:
- Sifting the raw materials
- Blending API with SLS in an amount of acetone
- Addition of internal phase excipients and the rest amount of acetone and blending.
- Drying the wet mass at 40°C
- Lubrication with Magnesium stearate and
- Compression

**Table 4: Dissolution profile of formulations 5 to 8**

| **Time interval (min)** | **% Dissolved** | | | |
|---|---|---|---|---|
| | **5** | **6** | **7** | **8** |
| 5 | 47,52 | 69,36 | 74,56 | 78,24 |
| 10 | 59,43 | 73,36 | 82,63 | 85,53 |
| 15 | 65,74 | 77,41 | 88,76 | 88,76 |
| 20 | 69,69 | 79,65 | 90,89 | 91,77 |
| 30 | 72,36 | 85,96 | 91,36 | 91,03 |
| 45 | 77,13 | 89,87 | 92,45 | 91,33 |

Formulations 6 to 8 having an amount of surfactant from 4% to 8% achieve the required release profile. Comparable results were obtained with the use of Soluplus® as the surfactant.

### Example 3:

Various modifications of formulation were also investigated in order to see how they affect the dissolution profile. In formulation trial 7 of example 2, Aerosil® was added in the external phase for better granule flow and the amount of disintegrant was increased to 8% by weight and split equally between internal and external phase. The amounts of other excipients were modified accordingly.

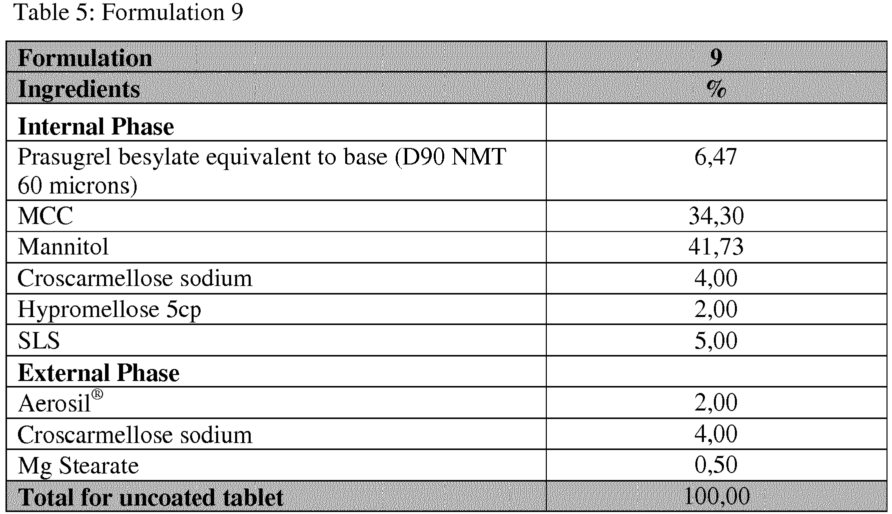

The manufacturing process of Formulation Trial 9 above was a wet granulation similar as in example 2.

**Table 6: Dissolution profile of formulation 9**

| **Time interval (min)** | **% Dissolved** |
|---|---|
| | **9** |
| 5 | 73,53 |
| 10 | 83,56 |
| 15 | 87,60 |
| 20 | 90,32 |
| 30 | 92,61 |
| 45 | 92,78 |

The release profile of formulation 9 is acceptable and comparable to that of formulations 6 to 8 of example 2. The only improvement observed was related to the disintegration time of the tablets that was reduced to below than 2 minutes.

### Example 4:

The formulation of example 3 was further evaluated with respect to change in manufacturing process and reduction of particle size of Prasugrel besylate. Three new formulation trials were prepared with the same qualitative and quantitative composition as that in table 5 (example 3). All were prepared by direct compression manufacturing process: sifting and blending all ingredients of external phase; mixing with Aerosil®; mixing with magnesium stearate and tableting

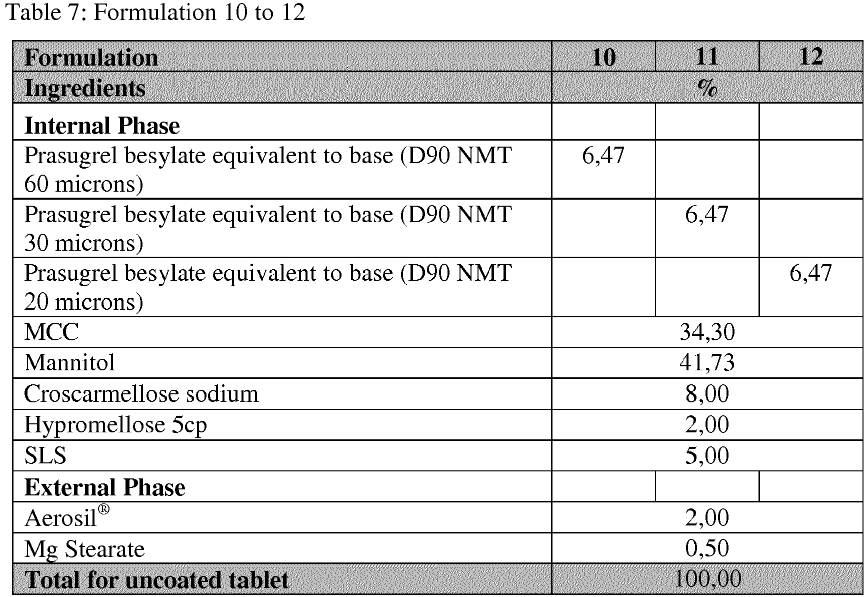

The dissolution profiles of formulations 10 to 12 are presented below.

**Table 8: Dissolution profile of formulations 10 to 12**

| **Time interval (min)** | **% Dissolved** | | |
|---|---|---|---|
| | **10** | **11** | **12** |
| 5 | 72,44 | 71,20 | 74,68 |
| 10 | 85,96 | 85,02 | 86,36 |
| 15 | 87,01 | 88,52 | 88,88 |
| 20 | 89,32 | 88,98 | 89,67 |
| 30 | 90,45 | 89,70 | 90,15 |
| 45 | 92,12 | 89,83 | 91,23 |

No significant difference was observed with particle size reduction and manufacturing process change.

Tablets resulted from the present invention were placed in chambers under normal, intermediate and accelerated conditions and were examined in appropriate time points in order to control their stability. The results were within limits proving a stable formulation during storage time.

## Claims

1. A pharmaceutical composition comprising prasugrel besylate having particle size of D₉₀ below 60µm, as measured by laser light diffraction and a surfactant in amount 4% to 8% by weight wherein the surfactant is selected from sodium lauryl sulfate and graft copolymer of polyethylene glycol / polyvinyl acetate / polyvinyl caprolactam.

2. A pharmaceutical composition according to claim 1, wherein Prasugrel besylate is in crystalline form.

3. A pharmaceutical composition according to claim 1, wherein Prasugrel besylate is in crystalline form P.

4. A pharmaceutical composition according to claim 1, wherein Prasugrel besylate is in crystalline form M.

5. A pharmaceutical composition according to claim 1, further comprises mannitol, microcrystalline cellulose, croscarmellose sodium, hypromellose, colloidal silicon dioxide and magnesium stearate.

6. A pharmaceutical composition according to claim 1 that comprises a filmcoating over the tablet core.

7. A process for preparing a pharmaceutical composition according to claim 1 comprising the steps of:
- Sifting the raw materials;
- Blending Prasugrel besylate with a surfactant selected from sodium lauryl sulfate and graft copolymer of polyethylene glycol / polyvinyl acetate / polyvinyl caprolactam in the presence of an amount of acetone or ethanol;
- Adding the internal phase excipients such as filler, disintegrant, diluent and the remaining amount of acetone and blending;
- Drying the wet mass at 40°C;
- Mixing with external phase excipients such as disintegrant and glidant;
- Lubrication with Magnesium stearate and
- Compression into tablet and optionally film coating the tablet core.

8. A process for preparing a pharmaceutical composition according to claim 1 comprising the steps of:
- Sifting the raw materials;
- Blending Prasugrel besylate with a surfactant selected from sodium lauryl sulfate and graft copolymer of polyethylene glycol / polyvinyl acetate / polyvinyl caprolactam;
- Adding the internal phase excipients such as filler, disintegrant, diluent and blending;
- Mixing with external phase excipients such as disintegrant and glidant;
- Lubrication with magnesium stearate and
- Compression into tablet and optionally film coating the tablet core.

## Patentansprüche

1. Pharmazeutische Zusammensetzung bestehend aus Prasugrel besilat mit einer Partikelgröße für den Wert D90 unter 60µm, wie diese mittels Laser-Streulichtspektrometers berechnet wird, sowie aus einem Tensid in einem Gewichtprozent zwischen 4% bis 8%, wobei das Tensid aus Natriumlaurylsulfat und Pfropf-Copolymer von Polyethylenglycol / Polyvinylacetat / Polyvinylcaprolactam ausgewählt wird.

2. Pharmazeutische Zusammensetzung nach Anspruch 1, **dadurch gekennzeichnet, dass** Prasugrel besilat in kristalliner Form vorliegt.

3. Pharmazeutische Zusammensetzung nach Anspruch 1, **dadurch gekennzeichnet, dass** Prasugrel besilat in kristalliner P-Form vorliegt.

4. Pharmazeutische Zusammensetzung nach Anspruch 1, **dadurch gekennzeichnet, dass** Prasugrel besilat in kristalliner M-Form vorliegt.

5. Pharmazeutische Zusammensetzung nach Anspruch 1, die weiterhin Mannitol, mikrokrystalline Cellulose, Croscarmellose-Natrium, Hydroxypropylmethylcellulose, kolloidales Siliciumdioxid und Magnesiumstearat umfasst.

6. Pharmazeutische Zusammensetzung nach Anspruch 1, die einen Filmüberzug des Tablettenkerns umfasst.

7. Verfahren für die Zubereitung einer pharmazeutischen Zusammensetzung nach Anspruch 1, welches aus (den) nachstehenden Schritten besteht:
- Rohstoffe sieben;
- Prasugrel besilat mit einem Tensid, welches aus Natriumlaurylsulfat und Pfropf-Copolymer von Polyethylenglycol / Polyvinylacetat / Polyvinylcaprolactam ausgewählt wird, mittels einer Menge von Aceton oder Ethanol vermischen.
- Die Hilfsstoffe der internen Phase, wie z.B. Füllstoff, Spreng-/Zerfallsmittel, Streckmittel, und die restliche Menge des Acetons hinzufügen und vermischen;
- Die feuchte Masse bei 40°C trocknen;
- Die Mischung mit den Hilfsstoffen der externen Phase, wie z.B. Spreng-/Zerfallsmittel und Gleitmittel vermischen;
- Mit Magnesiumstearat schmieren; und dann
- Das Granulat durch Kompression tablettieren und optional den Tablettenkern mit Film überziehen.

8. Verfahren für die Zubereitung einer pharmazeutischen Zusammensetzung nach Anspruch 1, welches aus den nachstehenden Schritten besteht:
- Rohstoffe vermischen;
- Prasugrel besilat mit einem Tensid, welches aus Natriumlaurylsulfat und Pfropf-Copolymer von Polyethylenglycol / Polyvinylacetat / Polyvinylcaprolactam ausgewählt wird, vermischen.
- Die Hilfsstoffe der internen Phase, wie z.B. Füllstoff, Spreng-/Zerfallsmittel, Streckmittel, hinzufügen und vermischen;
- Die Mischung mit den Hilfsstoffen der externen Phase, wie z.B. Spreng-/Zerfallsmittel und Gleitmittel vermischen;
- Mit Magnesiumstearat schmieren; und
- Das Granulat durch Kompression tablettieren und optional den Tablettenkern mit Film überziehen.

## Revendications

1. Une composition pharmaceutique qui comprend du bésylate de prasugrel ayant une granulométrie D₉₀ inférieure à 60 µm, telle que mesurée par diffraction de la lumière laser et un tensioactif en une quantité de 4% à 8% de sa masse dans laquelle le tensioactif est choisi parmi le laurylsulfate de sodium et le copolymère greffé de polyéthylène glycol / l'acétate de polyvinyle / le caprolactame de polyvinyle.

2. Une composition pharmaceutique, selon la 1ère revendication, où le bésylate de prasugrel est sous forme cristalline.

3. Une composition pharmaceutique, selon la 1ère revendication, où le bésylate de prasugrel est sous forme cristalline P.

4. Une composition pharmaceutique, selon la 1ère revendication, où le bésylate de prasugrel est sous forme cristalline M.

5. Une composition pharmaceutique, selon la 1ère revendication, qui comprend, en outre, du mannitol, de la cellulose microcristalline, de la croscarmellose sodique, de l'hypromellose, du dioxyde de silicium colloïdal et du stéarate de magnésium.

6. Une composition pharmaceutique, selon la 1ère revendication, qui comprend, en outre, le noyau enrobé.

7. Un procédé de préparation d'une composition pharmaceutique selon la 1ère revendication, qui comprend les étapes suivantes:
- Criblage des matières premières;
- Mélange du bésylate de prasugrel avec un tensioactif choisi parmi le laurylsulfate de sodium et le copolymère greffé de polyéthylène glycol / l'acétate de polyvinyle / le caprolactame de polyvinyle, en présence d'une quantité d'acétone ou d'éthanol;
- Ajout des excipients de phase interne tels que le liant, désintégrant, diluant et la quantité restante d'acétone et mélange;
- Séchage de la masse humide à 40°C;
- Mélange avec des excipients de phase externe tels que le désintégrant et le glissant;
- Lubrification au stéarate de magnésium et
- Mise en forme le comprimé et enrobage facultatif du noyau.

8. Un procédé de préparation d'une composition pharmaceutique selon la 1ère revendication, qui comprend les étapes suivantes:
- Criblage des matières premières;
- Mélange du bésylate de prasugrel avec un tensioactif choisi parmi le laurylsulfate de sodium et le copolymère greffé de polyéthylène glycol / l'acétate de polyvinyle / le caprolactame de polyvinyle;
- Ajout des excipients de phase interne tels que le liant, désintégrant, diluant et mélange;
- Mélange avec des excipients de phase externe tels que le désintégrant et le glissant;
- Lubrification au stéarate de magnésium et
- Mise en forme le comprimé et enrobage facultatif du noyau.
